# DEMANDE DE BREVET EUROPEEN

(11) **EP 4 682 509 A1**
(43) Date de publication de la demande: **21.01.2026**
(21) Numéro de dépôt: 25189924.1
(22) Date de dépôt: 16.07.2025
(51) Int. Cl.: G01N 21/17, G01N 21/84

(54) **METHODE PHOTOACOUSTIQUE DE CARACTERISATION D'UN VEGETAL**

(30) Priorité: 19.07.2024 FR 2407966
(71) Demandeur: Commissariat à l'Energie Atomique et aux Energies Alternatives, 75015 Paris (FR)
(72) Inventeur: JOURDE, Kévin, 38054 GRENOBLE Cedex 09 (FR); HANNART, Héloïse, 38054 GRENOBLE Cedex 09 (FR)
(74) Mandataire: Atout PI Laplace

(57) **Abrégé**

Méthode photoacoustique de caractérisation d'un végétal (10), la méthode comprenant l'application sur une surface de matière du végétal d'un rayonnement laser (105), un enregistrement par un microphone (175) d'ondes sonores apparaissant par effet photothermique dans une cavité photoacoustique (155) positionnée autour ladite surface du végétal, le rayonnement laser (105) ayant été appliqué à ladite surface à travers la cavité (155). La surface végétale est une surface d'une tige (ou racine ou pétiole) (11) du végétal, et la méthode comprend une insertion d'une section (16) de ladite tige (11) dans un support comprenant la cavité (155) équipée d'un capteur de microphone (175) et d'une fenêtre (170) pour transmission de la puissance du laser avec mise en place de moyens d'étanchéité (160, 162) autour de la tige (11) à chacune des deux extrémités de ladite section (16).

## Description

### Contexte technique

L'invention s'inscrit dans le domaine des appareils et méthodes pour l'exploration du vivant, notamment du monde végétal et plus précisément le suivi *in vivo* de l'activité des végétaux.

Elle exploite la photoacoustique - aussi appelée optoacoustique - c'est-à-dire l'observation des ondes mécaniques induites dans un corps illuminé par une radiation électromagnétique. Les phénomènes à la base de cette génération sont la photothermie : les ondes incidentes chauffent le corps, et la dilatation thermique : une élévation de température engendre une dilatation. Une lumière incidente variable dans le temps est utilisée pour créer une dilatation qui est suivie d'une rétractation.

La capacité à absorber la lumière dépend de la constitution de la matière et de l'onde électromagnétique. La photoacoustique est donc adaptée pour des manipulations de spectroscopie. On s'intéresse notamment à une approche par spectroscopie dans le domaine spectral du moyen infrarouge MIR. Cette gamme du spectre électromagnétique offre une signature spécifique des molécules. L'acquisition de spectres permet alors de remonter à la composition moléculaire des corps étudiés, et éventuellement de quantifier les espèces chimiques présentes.

Au cours des dernières années, des travaux ont tout d'abord porté sur le développement de capteurs de gaz miniatures reposant sur ce principe de la spectroscopie dans le moyen infra-rouge par transduction photoacoustique. Puis ces développements ont été appliqués à la caractérisation de liquides et de solides, et ont permis la mise au point par exemple d'un capteur capable de suivre le niveau de glycémie d'une personne de façon non-invasive.

La spectrométrie dans le moyen infrarouge sans transduction acoustique est aussi pratiquée dans le domaine de la biologie des végétaux. En effet, des travaux déjà réalisés sur les végétaux utilisent la micro-spectrométrie de type spectroscopie infrarouge à transformée de Fourier FTIR, qui permet d'établir des cartographies à l'échelle de la dizaine de microns de tissus de végétaux échantillonnés en lamelles ou réduits en poudre, par exemple pour l'analyse de la composition des parois des cellules, ce qui présente un intérêt pour la compréhension des communications intracellulaires et de façon concrète pour l'industrie du bois.

Des travaux de recherche ont également été menés pour développer l'utilisation de la photoacoustique pour l'étude des végétaux, toujours avec spectroscopie infrarouge. Il a été proposé de modifier la fréquence de modulation du faisceau optique pour déduire la composition chimique dans l'épaisseur d'échantillons de feuilles de végétaux.

Il a été proposé l'utilisation de cavités ouvertes apposées sur des feuilles de végétaux. On connait aussi de Pereira et al, Meas Sci. Technol. 3, 1992, 931, ainsi que de Mesquita et al. Instrumentation science and technology, vol 34, 33, 2006 de tels agencements pour l'étude in vivo de la photosynthèse, qui impliquent de placer une cavité ouverte en plaquant son embouchure contre une feuille de végétal, ce qui a pour conséquence de fermer la cavité. La feuille est, sur son autre face, exposée à une lumière visible (lumière blanche et lumière monochromatique à 680 nm ou 650 nm), guidée par fibre optique. La cavité comprend un microphone electret. Ces expériences, réalisées avec des longueurs d'onde visibles, visent à suivre in vivo l'activité de photosynthèse. Cependant la mesure est invasive (la feuille est recouverte) et ne permet donc pas un suivi prolongé sans impact sur l'activité du végétal.

Dans l'article Helfter C. et al. (2007) Tree Physiology, vol 27, 169, on observe la réponse d'une tige de végétal intègre à une source thermique en infrarouge proche (812 nm) grâce à une caméra infrarouge. L'approche est non invasive mais la caractérisation ne donne pas accès à des informations chimiques.

Il a aussi été proposé d'examiner des tissus vivants dans l'infrarouge moyen, par mesure FTIR en réflectance totale atténué (ATR-FTIR), dans Zhang et al. 2023, Scientific Reports, vol 13. Les limites de la méthode présentée dans cet article est le suivi temporel qui ne peut pas être fin sans perturbation du végétal, et la restriction de la zone examinée aux premiers microns de l'échantillon.

Enfin, dans Gaoqiang L., et al., 2020, Spectrochimica Acta Part A, vol 228, une approche par spectrométrie dans l'infrarouge moyen par photoacoustique pour rechercher la composition chimique dans des échantillons de feuilles de végétaux a été présentée.

### Exposé de l'invention

Il est proposé maintenant, ci-après, de plaquer une cavité photoacoustique sur la tige d'un végétal pour son étude et son suivi.

Pour surmonter les insuffisances présentées par ces systèmes et méthodes de l'art antérieur, il est proposé une méthode photoacoustique de caractérisation d'un végétal, la méthode comprenant l'application sur une surface de matière du végétal d'un rayonnement laser, un enregistrement par un microphone d'ondes sonores apparaissant par effet photothermique dans une cavité photoacoustique positionnée autour ladite surface du végétal, le rayonnement laser ayant été appliqué à ladite surface à travers la cavité.

De plus, de manière remarquable, la surface végétale est une surface d'une tige, racine ou pétiole du végétal, celui-ci étant intègre et typiquement vivant et en croissance, et la méthode comprend une insertion d'une section de ladite tige, racine ou pétiole dans un support comprenant la cavité équipée d'un capteur de microphone et d'une fenêtre pour transmission de la puissance du faisceau laser avec mise en place de moyens d'étanchéité autour de la tige, racine ou pétiole à chacune des deux extrémités de ladite section. Cette méthode est non invasive.

Ces principes permettent d'explorer la tige, racine ou pétiole d'un végétal, par exemple une tige de 2 à 3 mm de diamètre, et de sonder notamment le phloème et le xylème pendant l'activité du végétal, à diverses longueurs d'onde.

Diverses sources optiques pour la photo acoustique peuvent être utilisées. Un suivi sur une échelle de temps pertinente pour les évolutions du végétal permet de connaître les dynamiques associées et de mener des actions en conséquence.

Il est plus particulièrement proposé un système incluant un capteur pour suivre l'activité des végétaux par photothermie dans le moyen infrarouge.

Grâce au suivi par photothermie dans le moyen infrarouge MIR, on apporte la richesse de la spectroscopie dans le moyen infrarouge, la photothermie permettant de scanner l'échantillon dans son épaisseur - typiquement sur une centaine de microns, la profondeur exacte atteignable dépendant de la composition chimique du végétal étudié et de la gamme de longueurs d'ondes utilisées dans le MIR. Alors on peut obtenir des résolutions temporelles de suivi de l'ordre de la seconde, sur des échelles de temps long, par exemple plusieurs jours.

On réalise donc une mesure de spectroscopie dans le moyen infrarouge de la paroi de la tige, racine ou pétiole d'un végétal. Cette mesure se fait in vivo, est peu invasive, et peut être quasi-continue. Elle permet de détecter, avec une très forte probabilité, un signal spécifique à l'activité du végétal. Cette mesure a un fort potentiel pour aider à la compréhension des processus ayant lieu dans les végétaux, ce qui ouvre un large champ d'explorations fondamentales, et d'applications concrètes.

Préférentiellement, on mesure l'environnement simultanément, et on mesure en continu la puissance optique du laser. Dans les deux cas cela est effectué pour corriger les mesures, ou identifier des corrélations, qui peuvent être ou non non spécifiques à l'activité du végétal étudié

La modalité multifréquentielle de la photoacoustique permet de faire varier la profondeur de caractérisation de l'échantillon. Ainsi elle permet de scanner l'échantillon dans sa profondeur, et éventuellement de distinguer des processus se produisant à différentes profondeurs mais à l'intérieur de la couche la plus externe de 150 µm de la tige, racine ou pétiole.

Ainsi, de manière avantageuse et optionnelle :

- le support peut être composé d'un ensemble de deux mâchoires opposées formant étau et munies chacune d'un joint d'étanchéité ;

- le rayonnement laser peut être appliqué en onde continue avec une modulation d'amplitude à plusieurs fréquences ; on peut choisir ainsi des fréquences de modulation étalées par exemple de 100 à 1000 Hz, ce qui permet avec certaines de sonder des couches plus profondes de la tige, racine ou pétiole, et avec d'autre de sonder des couches plus superficielles ;

- le rayonnement laser peut être à une fréquence dans l'infrarouge moyen correspondant à une bande d'absorption d'une molécule d'intérêt pour le suivi de la croissance du végétal. Par exemple, on peut s'intéresser au glucose à 1036 cm⁻¹. Le rayonnement laser peut aussi être composé d'un ensemble de sources lasers à différentes longueurs d'onde optiques pertinentes pour l'analyse du végétal

- l'enregistrement peut être mené pendant une durée de l'ordre de 1 seconde, avec un échantillonnage à une fréquence de l'ordre de 100 kHz, mais de manière générale on enregistre un spectre photoacoustique (avec plusieurs fréquences de modulation) avec un ou plusieurs lasers, la façon dont ce spectre est enregistré étant adaptée au cas d'étude. Le spectre peut être enregistré en faisant plusieurs mesures à différentes fréquences de modulation (la durée étant alors dépendante du rapport signal à bruit désiré), ou en mode impulsionnel.

- la méthode peut consister à suivre le végétal de manière continue par des répétitions régulières de l'application du laser et de la mesure subséquente associée pendant au moins une journée et une nuit.

- la mesure peut être démodulée pour identifier une phase et un module, et ainsi réaliser un spectre photoacoustique.

- le végétal peut être vivant et est peu affecté par la réalisation de la mesure - c'est un grand avantage de la méthode : il n'y a pas de perturbation de l'activité de respiration ou de photosynthèse, et la mesure peut néanmoins être effectuée de manière très prolongée, de manière non invasive.

L'invention consiste aussi en un équipement photoacoustique pour caractérisation d'un végétal, l'équipement comprenant des moyens d'application d'un rayonnement laser, une cavité photoacoustique configurée pour que le rayonnement laser puisse être appliqué à travers la cavité, et des moyens d'enregistrement par un microphone d'ondes sonores apparaissant par effet photothermique dans la cavité photoacoustique.

L'équipement comprend de plus un support comprenant la cavité, le support comprenant aussi un espace d'insertion pour une section de tige, racine ou pétiole de végétal et des moyens d'étanchéité pour la cavité à chacune des deux extrémités de ladite section. Les joints d'étanchéité sont de préférence ajustés spécifiquement aux dimensions de la tige, racine ou pétiole testée. Les moyens d'application d'un rayonnement laser peuvent de plus comprendre un laser accordable, ou plusieurs lasers monochromatiques optiquement couplés.

On met ainsi au point un système installé à demeure sur le végétal sans impacter son développement

Les applications sont nombreuses, en lien notamment avec la crise climatique et la nécessité de suivre l'activité des végétaux pour aider le secteur agro-alimentaire à la compréhension de l'état de sa production et à l'anticipation de l'adaptation des végétaux aux changement environnementaux qu'ils vont vivre, ainsi qu'au probable futur manque de ressources disponibles.

Optionnellement, le support est composé d'un ensemble de deux mâchoires opposées formant étau et munies chacune d'un joint d'étanchéité circulaire aminci pour former avec le joint opposé un ensemble de deux passages opposés de part et d'autre de la cavité pour la mise en place de la tige, racine ou pétiole, avec étanchéité autour de la tige, racine ou pétiole.

### Brève description des figures

La figure 1 montre un schéma du dispositif de l'invention.
Les figures 2 à 5 montrent des vues tridimensionnelles d'un mode de réalisation du dispositif selon l'invention.
La figure 6 montre une tige de végétal et les espaces de celle-ci qui sont explorés grâce aux principes de l'invention.
Les figures 7 et 8 présentent des mesures obtenues avec le dispositif selon l'invention.

### Description en lien avec les dessins

On souhaite suivre la dynamique de croissance d'un végétal, par exemple la dynamique du transport et du stockage des sucres, et donc prévoir l'efficacité de celle-ci, pour par exemple que le secteur agricole puisse avoir une production de meilleure qualité nutritive tout en minimisant les apports extérieurs.

On souhaite aussi détecter la présence de produits chimiques liés à l'évolution de l'air ou des sols, le végétal étant une sorte de concentrateur, mis à profit pour la détection de variations subtiles de l'environnement. Dans le détail, on souhaite suivre temporellement le composé chimique pertinent dans le végétal, en fonction du temps. Pour cela, on exploite le spectre d'absorption associé à ce composé, ainsi que les spectres des composés qui risquent de générer un signal photoacoustique équivalent. Pour la ou les longueurs d'ondes qui paraissent les plus adaptées à la détection du composé, on évalue l'amplitude du signal associé aux variations de sa concentration en écartant les variations de signal lié aux variations de concentration des éventuels composés interférents.

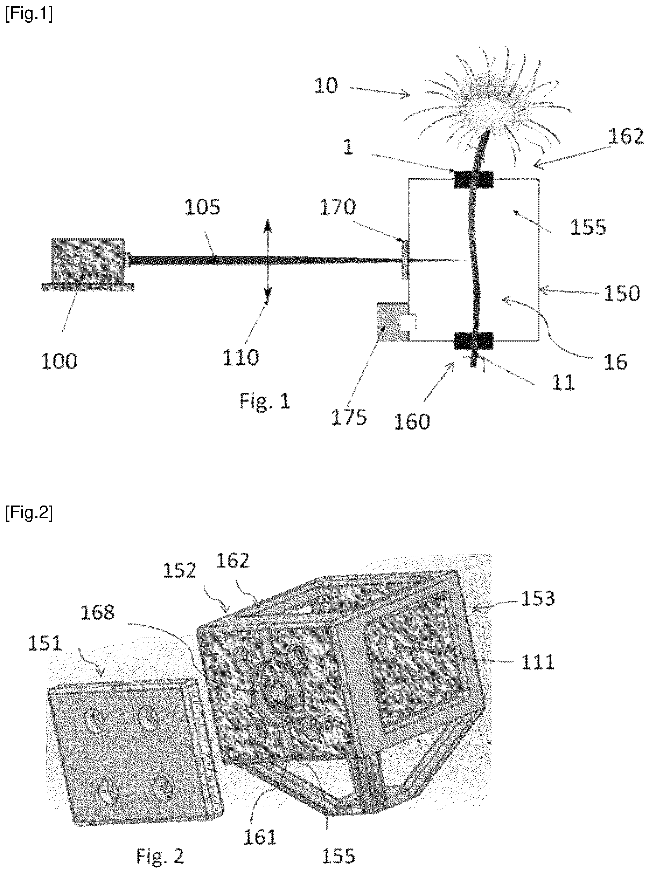

[Fig. 1] La figure 1 montre un montage mettant en œuvre les principes de l'invention pour le suivi de la plante 10.

Un laser 100 qui est un laser à cascade quantique émettant dans le moyen infrarouge produit un faisceau laser 105 qui est traité par une lentille convergente 110.

La source optique est un laser à cascade quantique QCL émettant dans le moyen infrarouge, dans une gamme spectrale restreinte, ou dans une gamme plus large avec possibilité d'accorder la source en longueur d'onde. Un cas particulier est le cas d"un laser monochromatique de type QCL DFB - Distributed FeedBack laser ou laser à rétroaction répartie. Un autre exemple est un laser QCL à cavité externe. On peut également avoir plusieurs lasers QCL et/ou DFB couplés par un système optique de type coupleur optique - puis allumés par intermittence.

Un boîtier étanche 150 définit une cavité 155, disposant de deux ouvertures 160 et 162 se faisant face sur deux parois opposées du boitier étanche 150. Le boîtier étanche 150 est formé en deux parties (non visibles sur la figure) mobiles l'une par rapport à l'autre et se joignant pour former la coque de la cavité et chacune des deux ouvertures 160 et 162. L'une des deux parties comprend une fenêtre 170 transparente aux ondes de la gamme du moyen infrarouge, par exemple une fenêtre en silicium ou en germanium avec un traitement antireflet adapté. L'une des deux parties comprend, tourné vers sa convexité, un microphone 175 (monté dans une carte électronique) pour effectuer les mesures de photoacoustique dans la cavité et disposant d'une alimentation électrique depuis l'extérieur du boitier et d'un moyen de communication des données captées vers un ordinateur extérieur. La partie du boitier qui accueille le microphone 175 et sa carte électronique peut être celle qui accueille la fenêtre 170, comme cela est montré sur la figure, mais il est aussi possible que ce soit l'autre.

La plante 10 dispose d'une tige 11 séparant des parties basses, comme typiquement les racines d'une part, et des parties hautes comme typiquement les feuilles, fruits et fleurs d'autre part. La tige est placée, pour le suivi de la plante 10, de façon à traverser les ouvertures 160 et 162 avant que les deux parties mobiles ne soient jointes pour former la cavité. Des moyens d'étanchéité sont de plus placés autour de la tige 11, dans chacune des ouvertures 160 et 162, de manière à assurer une étanchéité autour de celle-ci. Entre les deux ouvertures 160 et 162, c'est une section 16 de la tige qui s'étend - il convient qu'elle soit plus longue que la hauteur séparant les ouvertures 160 et 162. La mesure est réalisable, outre sur une tige, sur n'importe quel élément de forme cylindrique du végétal : tige, pétiole ou racine.

Le faisceau laser 105 est dirigé et la lentille 110 est placée pour que la puissance laser traverse la fenêtre 170 et soit focalisée sur la tige 11, dans la cavité 155.

On mesure la réaction photoacoustique du végétal pendant une longue période, et on mesure l'environnement simultanément, et on mesure également en continu la puissance optique du laser. On corrige les mesures de la réaction du végétal en fonction de l'environnement et du laser, et on recherche des corrélations. On corriger aussi les apports fournis au végétal pour améliorer le rendement de la culture.

[Fig. 2] Les figures suivantes montrent plusieurs vues tridimensionnelles d'un dispositif photothermique pour détection acoustique indirecte (l'onde acoustique est transmise par le gaz de la cavité, en l'occurrence de l'air jusqu'au microphone), conforme aux principes de l'invention.

La cavité photoacoustique est placée comme un étau - clampée - sur la tige 11 du végétal et la tige est ainsi dans la cavité. La cavité est étanchéifiée par des moyens adaptés, ayant un impact faible sur le végétal. Par exemple les moyens d'étanchéité sont des joints en silicone conçus sur mesure en fonction du diamètre des tiges à analyser. Ces joints en silicone permettent de sceller la cellule photoacoustique sans endommager le végétal. Le microphone détecte l'onde acoustique induite par l'effet photothermique dans la cavité photoacoustique.

Le boîtier étanche 150 est composé de deux mâchoires 151 et 152 formant chacune une des parties de boitier évoqué plus haut. En figure 2, les deux mâchoires sont placées en vis-à-vis l'une de l'autre, sans être au contact l'une de l'autre.

La mâchoire 151 a une forme générale de plaque rectangulaire dont l'une des faces (non visible en figure 2) porte des évidements aux fins de former d'une part la cavité 155 et d'autre part les ouvertures 160 et 162. L'autre face (visible en figure 2 et qui peut être qualifiée de face arrière) est moins fonctionnalisée et ne sera pas commentée ici.

L'autre mâchoire, à savoir la mâchoire 152 a aussi une forme générale de plaque rectangulaire, plus épaisse que la plaque de la mâchoire 151, mais de mêmes dimensions latérales que le rectangle de la mâchoire 151, l'assemblage des deux mâchoires 151 et 152 étant fait en mettant en vis-à-vis les côtés de mêmes dimensions.

La mâchoire 152 a sur une face (visible en figure 2) des évidements aux fins de former, avec des évidements correspondants de l'autre mâchoire, la cavité 155 et les ouvertures 160 et 162. Sur son autre face (non visible en figure 2, et qui peut être qualifiée de face arrière), la mâchoire porte une structure déportée 153 assez volumineuse pour d'une part le traitement optique de la lumière du laser et d'autre part la fixation du boîtier par exemple avec un bras ou un pied.

La cavité 155 est un espace de faible volume par rapport à l'ensemble de la construction formée par les deux mâchoires. Elle est formée par des évidements présents dans les deux plaques, au centre des rectangles constituant les faces de ces plaques qui sont apposées l'une à l'autre une fois la cavité formée. Les évidements des deux mâchoires sont de taille différente : la cavité est plus développée d'un côté du plan d'accolement des deux mâchoires que de l'autre, en l'occurrence dans la mâchoire 152.

La cavité 155 est plus précisément un espace délimité par une paroi ayant la forme d'un cylindre de révolution d'axe perpendiculaire aux plans des deux plaques. Cette paroi est formée dans la mâchoire 152. La cavité est aussi délimitée par des fonds, l'un dans une mâchoire, l'autre opposé dans l'autre mâchoire.

Les ouvertures 160 et 162 sont définies quant à elles par des évidements correspondants dans les deux plaques ayant ensemble une forme de cylindre de révolution d'axe parallèle au plan des plaques, et parallèle plus précisément, dans la réalisation présentée à la figure, au petit côté des rectangles formant les mâchoires. Ces évidements formant les ouvertures 160 et 162 sont formés pour moitié dans une mâchoire et pour moitié dans l'autre mâchoire. Les deux ouvertures 160 et 162 ont le même axe et le même diamètre, la cavité 155 se trouvant à mi-chemin entre la première ouverture et la deuxième ouverture. Les ouvertures sont débouchantes de part et d'autre de l'interface entre les deux mâchoires.

La face de la mâchoire 152 comprend de plus un évidement annulaire autour de la paroi cylindrique formant la cavité 155. Il a son pendant dans la face de la mâchoire 151 contre laquelle la mâchoire 152 est apposée lors de la fermeture de la cavité, de sorte à former un espace annulaire 168 pour la mise en place d'un joint torique entre les deux mâchoires. L'espace annulaire 168 rencontre les ouvertures 161 et 162 et dans le prolongement de celles-ci la paroi cylindrique qui sépare la cavité 155 de l'espace annulaire 168 est percée par une découpe qui prolonge les ouvertures 161 et 162 de telle sorte que la tige du végétal puisse être placée dans les ouvertures 161 et 162 tout en s'étendant dans la cavité et en passant à deux reprises dans l'espace annulaire 168, de part et d'autre de la cavité 155.

La structure déportée 153 comprend de plus une ouverture 111 traversante pour le passage de la lumière laser qui est placée dans le prolongement de la cavité 155.

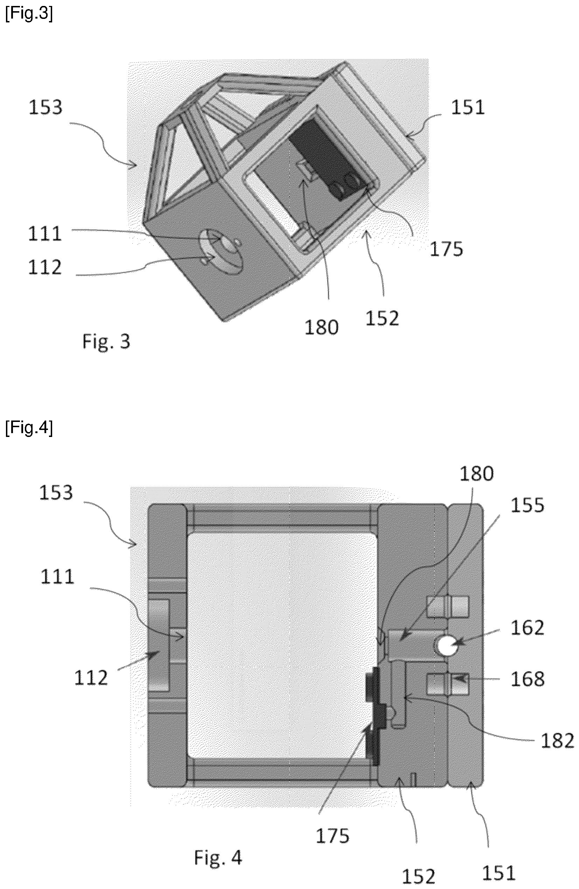

[Fig. 3] En figure 3, qui comme la figure 2 est une vue de trois-quarts, les deux mâchoires sont placées en vis-à-vis l'une de l'autre, et au contact l'une de l'autre, la cavité étant donc formée, et par conséquent invisible. On a représenté le capteur photoacoustique ou microphone 175 et sa carte électronique pour le suivi de l'activité des végétaux. Il s'agit d'un équipement encastré dans la mâchoire 152, et qui est affleurant sur la face arrière de celle-ci. On a également représenté une fenêtre 180 en matériau transparent aux longueurs d'onde du laser qui est présente, affleurant au milieu de la face arrière de la mâchoire 152, non loin de la carte électronique du microphone 175.

La structure déportée 153 présente visible sous l'angle de la vue un espace de fixation 112 pour la fente convergente. L'ouverture 111 traversante pour le passage de la lumière laser qui placée derrière cet espace de fixation 112. La fenêtre 180, l'espace de fixation 112 et l'ouverture 111 sont alignés.

[Fig. 4] En figure 4, la configuration de la figure 3 est représentée vue de côté, dans la direction des ouvertures 161 et 162 qui sont alignées perpendiculairement à la vue en section selon un plan passant par la cavité 155 dans un plan confondu avec l'axe optique.

Le microphone 175 est visible avec sa carte électronique tout comme l'ouverture 111 traversante pour le passage de la lumière laser et l'espace de fixation 112 pour la lentille convergente.

L'espace annulaire 168 apparait en deux fragments, de part et d'autre de la cavité 155. Il est symétrique de part et d'autre de l'interface entre les deux mâchoires (comme les ouvertures 161 et 162, mais contrairement à la cavité 155 qui s'étend plus dans la mâchoire 152.

La cavité 155 est visible : elle s'étend sur environ les quatre cinquièmes de l'épaisseur de la mâchoire 152, entre la surface d'accolement des deux mâchoires et la fenêtre 180 en matériau transparent aux longueurs d'onde du laser qui est présente au milieu de la face arrière de la mâchoire 152 dans le prolongement du cylindre constituant la cavité 155.

La mâchoire 152 comporte de plus dans son volume interne un conduit 182 qui permet à un son se propageant dans la cavité 155 d'être capté par le microphone 175. Le conduit 182 est débouchant dans la cavité 155 et se développe jusqu'à l'emplacement du capteur du microphone 175.

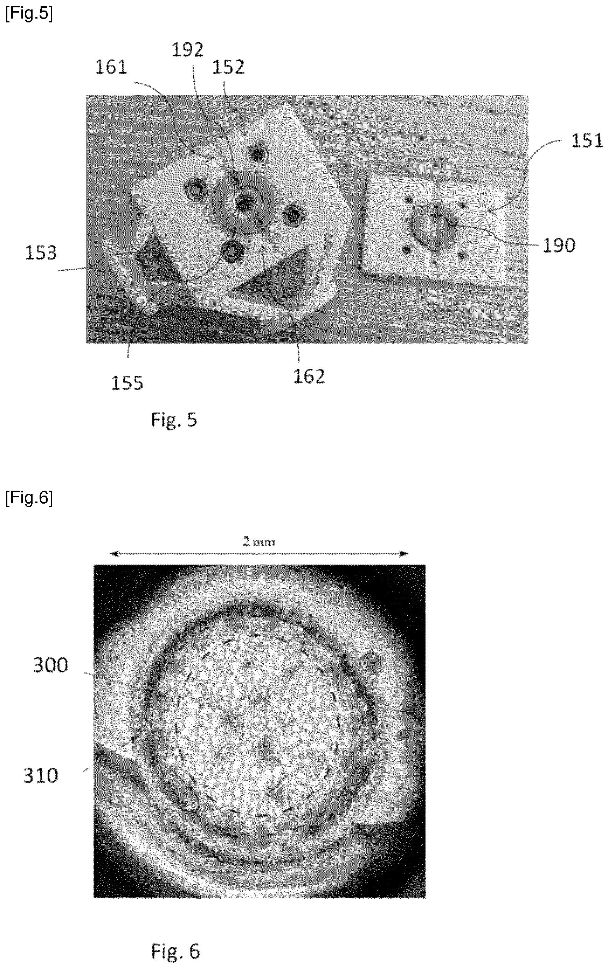

[Fig. 5] La figure 5 montre les deux mâchoires 151 et 152 dissociées l'une de l'autre. On ne voit la structure déportée 153 qu'en arrière-plan mais par contre, la cavité 155 est visible. Deux joints annulaires 190 et 192 sont présents dans les deux moitiés de l'évidement annulaire 168, respectivement à la surface de la mâchoire 151 et à la surface de la mâchoire 152. Ces joints sont en silicone et ont été formés avec un amincissement à placer dans le prolongement des ouvertures 161 et 162 pour ne pas compresser excessivement la tige de végétal.

[Fig. 6] La figure 6 est une photographie au microscope optique en lumière visible d'une coupe de la tige du végétal utilisé pendant des essais. L'image permet d'appréhender la zone sondée dans le moyen infrarouge.

Le végétal est une misère de nom biologique *tradescantia zebrina.* Le diamètre de la tige fait un peu moins de 2 mm. La zone annulaire dans laquelle se trouvent le phloème et le xylème est indiquée par la référence 300, et la profondeur de pénétration d'un faisceau laser à 1036 cm⁻¹ est indiquée par la référence 310 : on voit que le laser explore une importante partie externe de la zone 300.

L'eau liquide est un fort absorbant dans le moyen infrarouge, et il y a beaucoup d'eau dans les cellules. Il est donc courant qu'au premier ordre on commence par mesurer des variations d'eau.

L'eau représente aussi un frein direct à la possibilité d'examiner profondément dans le végétal notamment au-delà de 150 µm derrière la surface. Il est donc plus favorable de travailler sur des tiges et/ou racines jeunes et/ou fines, de diamètre inférieur à 1 mm.

A 1036 cm⁻¹, après l'eau, il est probable qu'on détecte la signature des composants des parois des cellules, principalement composées de cellulose et de lignine.

La modalité de mesure est adaptée pour faire une mesure relative et pour suivre l'évolution de la composition sondée en fonction du temps.

Il est en effet pertinent d'utiliser la méthode pour suivre la nature des fluides circulant dans le système vasculaire du végétal. Ce dernier étant localisé plutôt à la périphérie des végétaux, il est accessible à la photothermie.

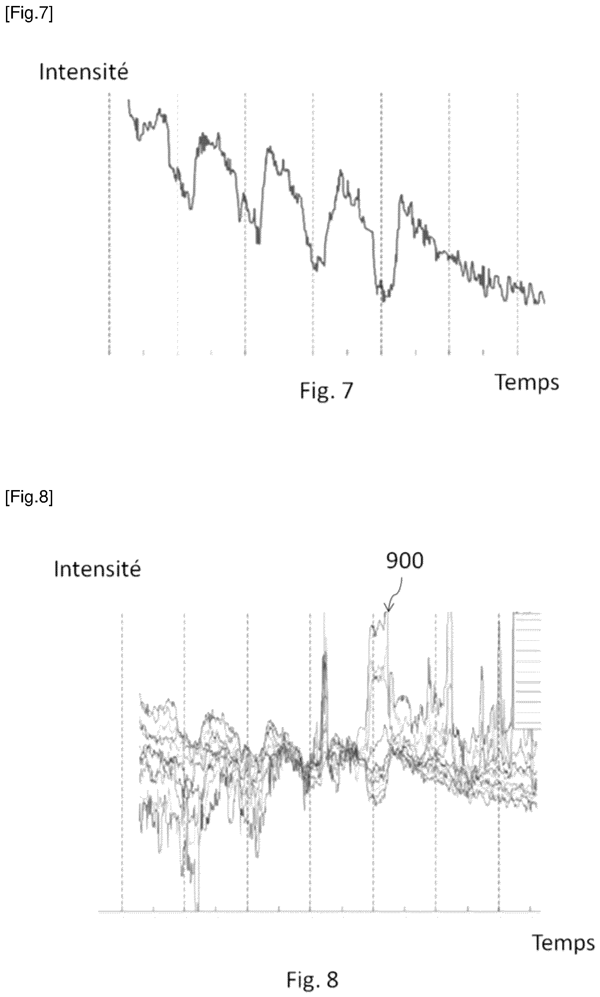

[Fig. 7] Une série de mesures a été réalisée avec le système décrit dans les sections précédentes, visible sur les figures 1 à 6, selon deux protocoles.

Le protocole 1 est une mesure continue pendant plusieurs jours avec un végétal installé dans le dispositif. La présence de la tige assure que la cavité est étanche, et le signal photoacoustique est capté par le microphone.

Le protocole 2 est une mesure continue dans les mêmes conditions que le protocole 1, mais sans végétal. La cellule photoacoustique est cependant bouchée au niveau du canal prévu pour la tige afin d'assurer la génération du signal photoacoustique. Le protocole 2 sert de référence afin de vérifier que les signaux détectés lors du protocole 1 sont induits par le végétal et non par une dérive du système, ou une sensibilité à l'évolution des paramètres environnementaux de l'expérience.

Les conditions expérimentales de la réalisation de ces essais sont l'utilisation d'un laser QCL laser Alpes, émission à 1036 cm-1, avec point de fonctionnement du laser courant moyen i0 = 0,710 A, une amplitude de modulation i1 = 0,10 A, et une température régulée à 25°C. La puissance du laser est estimée à 5 mW au niveau de la tige du végétal. Les fréquences de modulation sont 107, 134, 168, 210, 162, 328, 411, 514, 643, 805, 1007 Hz (et donc au nombre de 11 fréquences différentes), et le système met en place un scan composé de ces 11 fréquences. Le scan est répété toutes les 4 minutes.

Chaque mesure d'un scan consiste en une mesure d'une seconde avec échantillonnage à 100 kHz. Le signal est ensuite démodulé avec une fenêtre créneau à la fréquence de modulation du laser (démodulation dite 1f).

L'ensemble de la manipulation est réalisé à l'abri de la lumière naturelle, mais en présence, en journée, de lumière artificielle. La température est régulée. L'échantillon est une tige de végétal *tradescantia zebrina* pour le protocole 1. La lecture dure 165 heures pour le protocole 1 et 114 heures pour le protocole 2.

Des données brutes et traitées issues des deux expériences ont été examinées. Les données sont filtrées avec un filtre médian de largeur 1h20. On réduit le bruit de haute fréquence en augmentant le temps d'intégration de la détection synchrone. Un filtre médian est une solution adaptée pour ôter ce type de bruit. Puis les données sont débiaisées par soustraction d'une composante commune basse fréquence et d'une dérive lente, à l'échelle de plusieurs jours - définie par moyennage de l'ensemble des données aux différentes fréquences, et ajour d'un polynôme d'ordre 2. On s'intéresse également aux données filtrées et débiaisées sous la forme de spectrogrammes.

On installe un module de suivi des conditions environnementales du suivi. Les tendances temporelles observées ressemblent à des mesures de débit dans le xylème faites par d'autres moyens, et peuvent être reliées à un processus du végétal en rapport à l'exposition à la lumière. Ces tendances peuvent être l'augmentation de la vitesse de la circulation du fluide dans le xylème, et alors le changement des propriétés thermiques du végétal au cours de la journée, ou l'augmentation du volume d'eau ou de la concentration d'éléments absorbants dans le champ de vision du laser, là encore au cours de la journée.

La figure 7 montre l'évolution d'une partie du signal photoacoustique (ici la phase à 107 Hz en unité arbitraire et après filtration et débiaisement) en fonction du temps pendant une expérience d'une semaine (les sept jours de la semaine sont marqués par des traits pointillés verticaux). On observe des cycles journaliers qui s'interrompent le weekend lorsque la plante n'est plus exposée à la lumière.

[Fig. 8] La figure 8 montre l'évolution de différentes parties du signal photoacoustique (ici les phases aux 11 fréquences mentionnées précédemment) en fonction du temps pendant un suivi de huit jours (les jours de la semaine sont marqués par des traits pointillés verticaux).

On observe sur les phases et les modules une dynamique spatio-temporelle dans le végétal et en particulier un processus qui se produirait de l'intérieur vers l'extérieur (ou inversement) du végétal.

C'est particulièrement visible dans la zone temporelle 900 qui s'étale sur un peu plus d'une demi-journée, et au cours de laquelle on a un pic de signal qui se décale progressivement des hautes vers les basses fréquences.

Le système utilise, dans un mode de réalisation, une source accordable en longueur d'onde, afin de pouvoir caractériser spectralement l'échantillon et définir les longueurs d'onde adaptées suivant les applications visées.

L'invention a été décrite avec le mode d'émission de l'onde électromagnétique par onde continue à intensité modulée. Mais comme mentionné plus haut, elle peut être mise en œuvre avec une méthode impulsionnelle.

## Revendications

1. Méthode photoacoustique de caractérisation d'un végétal (10), la méthode comprenant l'application d'un rayonnement laser (105) sur une surface de matière du végétal, un enregistrement par un microphone (175) d'ondes sonores apparaissant par effet photothermique dans une cavité photoacoustique (155) positionnée autour ladite surface du végétal, le rayonnement laser (105) ayant été appliqué à ladite surface à travers la cavité (155), la méthode étant **caractérisée en ce que** la surface végétale est une surface d'une tige, racine ou pétiole (11) du végétal, et la méthode comprend une insertion d'une section (16) de ladite tige racine ou pétiole (11) dans un support comprenant la cavité (155) équipée d'un capteur de microphone (175) et d'une fenêtre (170) pour transmission de la puissance du laser avec mise en place de moyens d'étanchéité (160, 162) autour de la tige, racine ou pétiole (11) à chacune des deux extrémités de ladite section (16).

2. Méthode photoacoustique de caractérisation d'un végétal selon la revendication 1, **caractérisée en ce que** le support est composé d'un ensemble de deux mâchoires (151, 152) opposées formant étau et munies chacune d'un joint d'étanchéité.

3. Méthode photoacoustique de caractérisation d'un végétal selon la revendication 1 ou la revendication 2, **caractérisée en ce que** le rayonnement laser (105) est appliqué en onde continue avec une modulation d'amplitude à plusieurs fréquences.

4. Méthode photoacoustique de caractérisation d'un végétal selon l'une des revendications 1 à 3, **caractérisée en ce que** le rayonnement laser (105) est à une fréquence dans l'infrarouge moyen correspondant à une bande d'absorption d'une molécule d'intérêt pour le suivi de la croissance du végétal.

5. Méthode photoacoustique de caractérisation d'un végétal selon l'une des revendications 1 à 4, **caractérisée en ce qu'**elle consiste à suivre le végétal de manière continue par des répétitions régulières de l'application du laser et de la mesure associée pendant au moins une journée et une nuit.

6. Méthode photoacoustique de caractérisation d'un végétal selon l'une des revendications 1 à 5, **caractérisée en ce que** la mesure est démodulée pour identifier une phase et un module.

7. Méthode photoacoustique de caractérisation d'un végétal selon l'une des revendications 1 à 6, **caractérisée en ce que** le végétal (10) est vivant.

8. Equipement photoacoustique pour caractérisation d'un végétal, l'équipement comprenant des moyens (100) d'application d'un rayonnement laser, une cavité photoacoustique (155) configurée pour que le rayonnement laser puisse être appliqué à travers la cavité, et des moyens d'enregistrement par un microphone (175) d'ondes sonores apparaissant par effet photothermique dans la cavité photoacoustique (155), **caractérisé en ce que** l'équipement comprend de plus un support comprenant la cavité, le support comprenant aussi un espace d'insertion pour une section de tige, racine ou pétiole de végétal et des moyens d'étanchéité (190, 192) pour la cavité à chacune des deux extrémités de ladite section.

9. Equipement photoacoustique pour caractérisation d'un végétal selon la revendication 8, **caractérisé en ce que** le support est composé d'un ensemble de deux mâchoires (151, 152) opposées formant étau et munies chacune d'un joint d'étanchéité circulaire pour former avec le joint opposé un ensemble de deux passages opposés de part et d'autre de la cavité avec étanchéité pour la mise en place de la tige, racine ou pétiole.

10. Equipement photoacoustique pour caractérisation d'un végétal selon la revendication 8 ou la revendication 9, **caractérisé en ce que** les moyens (100) d'application d'un rayonnement laser comprennent un laser accordable, ou plusieurs lasers monochromatiques optiquement couplés.
